**Europäisches Patentamt**

**European Patent Office**

**Office européen des brevets**

(19)

(11) Publication number: **0 153 847**
**B1**

(12) **EUROPEAN PATENT SPECIFICATION**

(45) Date of publication of patent specification: **29.06.88**

(51) Int. Cl.⁴: **A 61 B 17/36**

(21) Application number: **85301123.7**

(22) Date of filing: **20.02.85**

(54) Intravascular laser catheter.

(30) Priority: **23.02.84 US 582675**

(43) Date of publication of application:
**04.09.85 Bulletin 85/36**

(45) Publication of the grant of the patent:
**29.06.88 Bulletin 88/26**

(84) Designated Contracting States:
**AT BE CH DE FR GB IT LI NL**

(56) References cited:
**DE-A-2 053 301**
**DE-A-2 106 470**
**GB-A-2 017 506**

**OPTICAL FIBRE TELECOMMUNICATIONS,
Miller et al., 1981, pages 241-243, Academic
Press Inc.(London) Ltd., London, GB;**

(73) Proprietor: **SHILEY INCORPORATED**
**17600 Gillette**
**Irvine California (US)**

(72) Inventor: **Edelman, William**
**4873 Dogwood Avenue**
**Seal Beach California (US)**

(74) Representative: **Boydell, John Christopher et al**
**Stevens, Hewlett & Perkins 5 Quality Court**
**Chancery Lane**
**London, WC2A 1HZ (GB)**

Courier Press, Leamington Spa, England.

## Description

The present invention relates to apparatus for directing laser energy to vascular obstructions. More particularly it relates to apparatus for directing laser energy through an intravascular catheter to a fixed focal point in order to dissolve an occlusion in a blood vessel.

Current methods used to remove vascular obstructions such as fatty deposits, plaque, calcification, and emboletic clots in occluded vessels include drug treatment using lysing agents, resection of the occluded vessel, displacement with embolectomy catheters, and balloon angioplasty. In using the embolectomy catheter, the clot must be located and isolated and then surrounded and removed. Balloon angioplasty increases the diameter of an occluded vessel by inflating a balloon and applying pressure stepwise to the sides of the occlusion. This technique is not useful when the vessel is completely occluded. There remains a need for an efficient device to destroy such lesions *in situ* without adverse side effects, even in completely occluded vessels.

U.S. Patent No. 4,207,874 discloses a fibre-optic device with a coned head configuration at the distal end thereof. This configuration utilises the coned head to bend the direction of the laser beam, thereby concentrating it near the distal end of the device.

The present invention seeks to provide apparatus capable of directing laser energy to vascular obstructions in a controlled manner in order to dissolve and remove them.

According to the invention there is provided apparatus for directing laser energy at a fixed focal point in the body of a patient, said apparatus comprising a catheter having proximal and distal ends and at least one lumen therethrough, a bundle of solid optical fibres in an annular array about the outer wall of said catheter, said fibres having proximal and distal ends and being adapted at their proximal ends for association with an energy source to supply laser energy for transmission to the body of the patient, the distal end of said fibre bundle being bevelled at such an angle as to substantially focus said laser energy at a single focal point.

The optical fibres may be, for example, fused silica, quartz or glass, and preferably the fibre bundle is enclosed in an outer annular sheath. In a preferred embodiment of the invention, the annular sheath is bifurcated in the vicinity of the proximal end forming a first leg which is adapted to receive the fibres, and a second leg enclosing the catheter. The first leg may be provided with means for association with an energy source, while the second leg terminates in an injection port provided with means for injecting contrast media or a therapeutic fluid through the lumen, or is provided with means for insertion of a guide wire through the lumen.

As used herein, the term "to reduce a vascular obstruction", or the like, means to substantially reduce the size of the lesion. Preferably, of course, treatment is continued until essentially complete removal of the lesion has been achieved.

In order that the invention may be better understood, an embodiment thereof will now be described by way of example only and with reference to the accompanying drawings in which:—

Figure 1 is a side elevational view of an embodiment of a laser catheter according to the present invention;

Figure 2 is a cross-sectional view along the line 2—2 of Figure 1;

Figure 3 is a cross-sectional view along the line 3—3 of Figure 1; and

Figure 4 is a cross-sectional view along the line 4—4 of Figure 1.

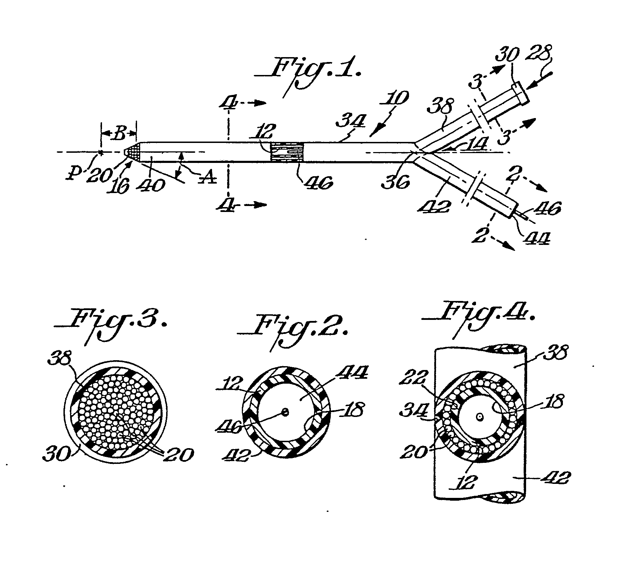

In Figures 1 to 4 is depicted an apparatus 10 for directing laser energy to a fixed focal point in an occluded blood vessel, thereby dissolving and removing the occlusion. Apparatus 10 includes catheter 12 having proximal and distal ends 14, 16 respectively, and having a lumen 18 passing therethrough. A bundle of solid optical fibres 20, each having a core and a clad of different refractive indices, is arranged in an annular array around the outer wall 22 of the catheter 12. Fibres 20 are adapted at their proximal end for association with a laser energy source 28 through a coupling 30. The optical fibres 20 may be glass or quartz, but are preferably fused silica.

The diameter of the optical fibres 20 is generally between about 0.0254 mm (0.001 inches) and 1.27 mm (0.050 inches), preferably 0.0254 mm (0.001 inches) to 0.508 mm (0.020 inches). The length of the fibres 20 is generally about 50 to 300 cm., preferably 100 to 150 cm. The number of fibres in the bundle, which is arranged in annular array around the outer wall 22, is typically between about 35 and 100, preferably 50 to 75.

In a preferred embodiment, apparatus 10 is provided with an outer annular sheath 34, preferably made from a flexible material, especially silicone rubber tubing, enclosing fibre bundle 20. Sheath 34 is bifurcated at 36 in the vicinity of the proximal end thereof, forming a first leg 38 through which are fed fibres 20 which transmit laser energy from source 28 to distal end 40 of the fibre bundle. Sheath 34 at bifurcation 36 also extends to second leg 42 enclosing catheter 12. Second leg 42 of sheath 34 terminates in an injection port 44 for insertion of guide wire 46 through lumen 18. The guide wire is typically radiopaque. Alternatively, injection port 44 may be provided with reservoir containing contrast medium or therapeutic fluid for injection through lumen 18. The therapeutic fluid may be, for example, saline to rinse the vessel, thrombogentic agents to dissolve clots, or vasodilators to enlarge the vessel.

In accordance with the present invention, fibre bundle 20 is bevelled at an angle at the distal end 40 so as to substantially focus laser energy transmitted from source 28 to focal point P. Bevelling of the fibre bundle 20 may be achieved by rotating the distal end about its axis while the leading edge is pressed against a rotating disk. The rotating disk material is such that the fibre bundle 20 is both abraded and polished simultaneously.

The angle A of the bevel of the fibre bundle 20 is selected such that it focuses the laser at a focal point P. The focal length B is related to the angle A of the bevel by the following formula:—

$$B = \left[ \frac{\sin(90° - \sin^{-1}((N_1/N_2)\ \sin\ \alpha) - \alpha)}{\sin(\sin^{-1}((N_1/N_2)\sin\ \alpha) - \alpha)} \right] a$$

where:—

$N_2$ = index of refraction of the optical fibre clad
$N_1$ = index of refraction of the optical fibre core
a = radius of the annular array of fibres from catheter axis to centre of fibre
$\alpha$ = bevel angle A
B = focal length

The catheter diameter and materials of contruction of the optical fibres are factors in the relationship between the bevel angle and focal length.

Thus, for example, when $N_2$ is 1.402, $N_1$ is 1.492 (as for a typical quartz fibre) and a is 0.889 mm (0.035 inches), the focal length depends on the bevel angle as shown in the following table:—

| (°)<br>α | B (in) mm |
|---|---|
| 0 | Infinity |
| 10 | (3.115)  79.125 |
| 30 | (0.945)  24.003 |
| 50 | (0.420)  10.668 |
| 70 | (0.105)  2.667 |

Accordingly, when a, $N_1$ and $N_2$ are given, a desired focal length can be obtained by appropriate selection of the corresponding bevel angle. For the reduction of vascular obstructions, a focal length of from about 2.54 mm (0.100 in) to about 5.08 mm (0.200 in) is preferred.

In use, the occlusion is located by angiography, or percutaneous puncture. The laser catheter is then advanced through an arteriotomy up to the occlusion. In peripheral arteries contrast media is injected through the catheter to locate the occlusion with a fluoroscope. With the distal tip of the annular array of fibres spaced from the occlusion by the fixed focal length of the device, the laser is then fired to destroy the occlusion. With coronary arteries the contrast media is injected through the catheter to locate the coronary arterial tree. A guide wire is threaded through the catheter into the selected arterial segment, until it comes into contact with the occlusion, the radiopaque nature of the guide wire facilitating the detection of position of the occlusion by fluoroscopy.

The catheter is then threaded over the guide wire to a position proximal to the distal tip of the guide wire, which is substantially equal to the focal length B of the catheter as determined by the bevelled angle A.

The fixed focal point laser catheter of the present invention eliminates many of the problems of previously used methods for removal of vascular obstructions, and provides a disposable, relatively simple system, which substantially focuses the laser energy at a single fixed, preset point, thereby eliminating the risk of accidental perforation of the vessel. The novel device of this invention has the further advantage of being effective even in a completely occluded vessel. In addition, it is of relatively uncomplicated design, offers easy manipulation and insertion, and is adapted for use with known laser energy sources.

**Claims**

1. Apparatus for directing laser energy at a fixed focal point in the body of a patient, said apparatus comprising:—
   a catheter having proximal and distal ends and at least one lumen therethrough;
   a bundle of solid optical fibres in an annular array about the outer wall of said catheter, said fibres having proximal and distal ends and being adapted at their proximal ends for association with an energy source to supply laser energy for transmission to the body of the patient;
   the distal end of said fibre bundle being bevelled at such an angle as to substantially focus said laser energy at a single focal point.

2. The apparatus of claim 1 wherein said fibres are made of fused silica.

3. The apparatus of claim 1 wherein said fibres are made of glass or quartz.

4. The apparatus of claim 1 including an outer annular sheath enclosing said fibre bundle. ·

5. The apparatus of claim 4 wherein said sheath is bifurcated in the vicinity of its proximal end, forming a first leg adapted to receive said fibres and a second leg enclosing said catheter.

6. The apparatus of claim 5 wherein said second leg terminates in an injection port.

7. The apparatus of claim 6 wherein said injection port is provided with means for injection of contrast media through said lumen.

8. The apparatus of claim 6 wherein said injection port is provided with means for injection of a therapeutic fluid.

9. The apparatus of claim 6 wherein said injection port is provided with means for insertion of a guide wire through said lumen.

10. Apparatus for directing laser energy to an occlusion at a fixed focal point in a blood vessel, said apparatus comprising:—
   a catheter having proximal and distal ends and a lumen therethrough;
   a bundle of solid optical fibres in an annular array about the outer wall of said catheter, said fibres having proximal and distal ends, the distal end of said fibre bundle being bevelled at such an angle as to substantially focus said laser energy at a single focal point, said fibres being adapted at their proximal end for association with an energy source to supply laser energy for transmission to said occlusion; and

an outer annular sheath enclosing said fibre bundle, said sheath being bifurcated in the vicinity of the proximal end thereof, thereby forming a first leg adapted to receive said fibres and a second leg terminating in an injection port provided with means for insertion through said lumen of a guide wire.

## Patentansprüche

1. Vorrichtung zum Richten von Laserenergie auf einen festen Brennpunkt im Körper eines Patienten, mit:
einem Katheter, der proximale und distale Enden sowie wenigstens ein hindurchgehendes Lumen hat;
einem Bündel von vollem otischen Fasern in einer Ringanordnung um die Außenwand des Katheters herum, wobei die Fasern proximale und distale Enden haben und an ihrem proximalen Enden zur Verbindung mit einer Energiequelle zur Zuführung von Laserenergie für die übertragung zum Körper des Patienten eingerichtet sind;
wobei das distale Ende des Faserbündels unter einem solchen Winkel abgeschrägt ist, um die Laserenergie im wesentlichen in einem einzigen Brennpunkt zu fokussieren.

2. Vorrichtung nach Anspruch 1, bei der die Fasern aus Kieselerdeschmelzglas bestehen.

3. Vorrichtung nach Anspruch 1, bei der die Fasern aus Glas oder Quarz bestehen.

4. Vorrichtung nach Anspruch 1, mit einer äußeren ringförmigen Hülle, die das Faserbündel umschließt.

5. Vorrichtung nach Anspruch 4, bei der die Hülle benachbart ihrem proximalen Ende gebelartig geteilt ist, wobei ein erster, zur Aufnahme der Fasern eingerichteter Schenkel und ein zweiter, den Katheter umschließender Schenkel gebildet sind.

6. Vorrichtung nach Anspruch 5, bei der der zweite Schenkel in einer Einspritzöffnung endigt.

7. Vorrichtung nach Anspruch 6, bei der die Einspritzöffnung mit Mitteln zum Einspritzen von Kontrastmitteln durch das Lumen versehen ist.

8. Vorrichtung nach Anspruch 6, bei der die Einspritzöffnung mit Mitteln zum Einspritzen eines therapeutischen Fluids versehen ist.

9. Vorrichtung nach Anspruch 6, bei der die Einspritzöffnung mit Mitteln zum Einführen eines Führungsdrahtes durch das Lumen versehen ist.

10. Vorrichtung zum Richten von Laserenergie auf einen Verschluß in einem festen Brennpunkt in einem Blutgefäß, mit:
einem Katheter, der proximale und distale Enden sowie ein hindurchgehendes Lumen hat;
einem Bündel von vollem otischen Fasern in einer Ringanordnung um die Außenwand des Katheters herum, wobei die Fasern proximale und distale Enden haben, wobei das distale Ende des Faserbündels unter einem solchen Winkel abgeschrägt ist, um die Laserenergie im wesentlichen in einem einzigen Brennpunkt zu fokussieren, welche Fasern an ihrem proximalen Ende zur Verbindung mit einer Energiequelle zur Zuführung von Laserenergie für die übertragung zum Verschluß eingerichtet sind; und

einer äußeren ringförmigen Hülle, die das Faserbündel umschließt, wobei die Hülle benachbart ihrem proximalen Ende gebelartig geteilt ist, wodurch ein erster, zur Aufnahme der Fasern eingerichteter Schenkel sowie ein zweiter Schenkel gebildet sind, der in einer Einspritzöffnung endigt, die mit Mitteln zum Einführen eines Führungsdrahtes durch das Lumen versehen ist.

## Revendications

1. Appareil destiné à diriger l'énergie d'un laser vers un point focal.fixe dans le corps d'un patient, ledit appareil comprenant:
un cathéter présentant une extrémité proximale et une extrémité distale, et au moins une ouverture le traversant;
un faisceau de fibres optiques pleines en une rangée annulaire autour de la paroi externe dudit cathéter, lesdites fibres présentant des extrémités proximales et distales, leurs extrémités proximales pouvant être reliées à un source d'énergie destinée à fournir l'énergie d'un laser afin de la transmettre dans le corps du patient;

l'extrémité distale dudit faisceau de fibres étant taillée en cône à un angle tel qu'il permette de focaliser ladite énergie du laser pratiquement en un seul point focal.

2. Appareil suivant la revendication 1, dans lequel lesdites fibres sont faites de verre de silice.

3. Appareil suivant la revendication 1, dans lequel lesdites fibres sont faites de verre ou de quartz.

4. Appareil suivant la revendication 1, comprenant une gaine annulaire externe entourant le faisceau de fibres.

5. Appareil suivant la revendication 4, dans lequel la gaine présente une bifurcation non loin de son extrémité proximale, formant un premier bras pouvant recevoir les fibres et un second bras renfermant le cathéter.

6. Appareil suivant la revendication 5, dans lequel le second bras se termine par un orifice d'injection.

7. Appareil suivant la revendication 6, dans lequel l'orifice d'injection est muni d'un système permettant l'injection d'un milieu de contraste à travers l'ouverture.

8. Appareil suivant la revendication 6, dans lequel l'orifice d'injection est muni d'un système permettant l'injection d'un fluide thérapeutique.

9. Appareil suivant la revendication 6, dans lequel l'orifice d'injection est muni d'un système permettant d'insérer un fil de guidage à travers l'ouverture.

10. Appareil permettant de diriger l'énergie d'un laser vers un obstacle situé à un point focal fixe dans un vaisseau sanguin, ledit appareil comprenant:

un cathéter présentant des extrémités proximale et distale et une ouverture le traversant;

un faisceau de fibres optiques pleines en une rangée annulaire autour de la paroi externe dudit cathéter lesdites fibres présentant des extrémités proximales et distales, l'extrémité distale dudit faisceau de fibres étant taillée en cône à un angle choisi de manière qu'il permette de focaliser ladite énergie du laser pratiquement en un seul point focal, et l'extrémité proximale desdites fibres pouvant être reliée à une source d'énergie destinée à fournir l'énergie d'un laser afin de la transmettre audit obstacle; et

une gaine annulaire externe entourant ledit faisceau de fibres, ladite gaine présentant une bifurcation non loin de son extrémité proximale, formant ainsi un premier bras pour recevoir lesdites fibres et un second bras se terminant par un orifice d'injection muni d'un système permettant l'insertion d'un fil de guidage à travers ladite ouverture.

*Fig.1.*

*Fig.3.*

*Fig.2.*

*Fig.4.*